# EUROPÄISCHE PATENTSCHRIFT

(11) **EP 4 501 367 B1**
(45) Veröffentlichungstag und Bekanntmachung des Hinweises auf die Patenterteilung: **10.06.2026**
(21) Anmeldenummer: 24220568.0
(22) Anmeldetag: 10.08.2020
(51) Int. Cl.: A61L 27/30, A61L 31/08

(54) **BESCHICHTUNG EINER STRUKTURIERTEN IMPLANTATOBERFLÄCHE**
COATING OF A STRUCTURED IMPLANT SURFACE
REVÊTEMENT D'UNE SURFACE STRUCTURÉE D'IMPLANT

(43) Veröffentlichungstag der Anmeldung: 05.02.2025
(62) Teilanmeldung aus: 20190262.4
(73) Patentinhaber: Waldemar Link GmbH & Co. KG, 22339 Hamburg (DE)
(72) Erfinder: LINK, Helmut D., 22397 Hamburg (DE); SPATH, Sebastian, 22297 Hamburg (DE); CSASZAR, Richard, 23795 Bad Segeberg (DE)
(74) Vertreter: Alatis

(56) Entgegenhaltungen:
- EP-A1- 2 444 108
- EP-A1- 3 566 725

## Beschreibung

### TECHNISCHES GEBIET

Die vorliegende Erfindung betrifft eine antimikrobielle Implantatkomponente mit einer beschichteten Oberflächenstruktur sowie ein Verfahren zum Beschichten so einer mit einer Oberflächenstruktur versehenen Implantatkomponente mit einer antimikrobiellen Beschichtung.

### HINTERGRUND DER ERFINDUNG

Bei der Integration einer Implantatkomponente in die anatomische Umgebung eines Implantationsortes von einem Patienten spielt die Implantatoberfläche eine wesentliche Rolle. Vor allem aus diesem Grund wird auf vielfältige Weise versucht, die Implantatoberfläche so bereitzustellen oder zu modifizieren, dass eine Implantatkomponente seine Aufgabe erfolgreich erfüllen kann.

Ein mögliches Ziel für die Auswahl einer bestimmten Implantatoberfläche kann zum Beispiel sein, dass die Implantatoberfläche das Einwachsen von Knochengewebe oder Weichteilgewebe am Implantationsort begünstigt. Insbesondere hierfür gibt es vielfältige Ansätze, um auf die Eigenschaften der Implantatoberfläche Einfluss zu nehmen. Hierzu gehören zum Beispiel die Auswahl des Materials, aus dem die Implantatoberfläche besteht, oder die Beschaffung der Oberflächenstruktur, die nach der Implantation mit dem Gewebe in Kontakt kommt.

Ein Ansatz zum Ausführen einer Implantatoberfläche besteht darin, das Material des Implantatkörpers mit einer definierten Oberflächenstruktur zu versehen, um so beispielsweise die Implantatoberfläche und damit die Kontaktfläche zum umliegenden Gewebe zu vergrößern. Hierfür wird die Implantatoberfläche selbst oder mit dem Auftrag zusätzlichen Materials strukturiert. Eine so erzeugte Implantatoberfläche kann ein Einwachsen des Gewebes und damit die Integration einer Implantatkomponente fördern.

Neben der Erzeugung einer Oberflächenstruktur kann eine Implantatoberfläche durch das Aufbringen einer Beschichtung modifiziert werden, um die Funktionalität der Implantatoberfläche zur Unterstützung des jeweiligen Behandlungsziels zu unterstützen. Handelt es sich bei der zu beschichtenden Implantatoberfläche um eine strukturierte Oberfläche, ergeben sich nach wie vor jedoch Herausforderungen für den Beschichtungsvorgang. Hierzu gehören zum Beispiel die Homogenität oder gleichmäßige Dicke der Beschichtung. Bedingt werden diese Herausforderungen insbesondere durch Kanten, Spitzen und Hinterschneidungen der Oberflächenstruktur.

Aus der EP3566725A1 ist eine Beschichtung für eine Implantatkomponente, insbesondere eine Komponente eines Wirbelsäulenimplantats, vorbekannt. Die Beschichtung ist eine keramische Titannitridbeschichtung, die neben einem At%-Anteil Ti und einem At%-Anteil N einen At%-Anteil von 5-30 At% Ag aufweist.

Aus der EP2444108A1 ist eine Prothesenkomponente einer Gelenkendoprothese vorbekannt, die eine Gleitfläche umfasst, die dazu ausgelegt ist, mit einer Gegengleitfläche einer anderen Prothesenkomponente ein Gleitgelenk zu bilden. Die Gleitfläche ist von einer auf den Körper der Prothesenkomponente aufgebrachten Gleitflächenbeschichtung gebildet, die eine antimikrobielle Wirkung hat und gegen Mikroorganismen im Bereich des Gleitgelenks wirkt.

### ZUSAMMENFASSUNG DER ERFINDUNG

Somit war es eine Aufgabe der vorliegenden Offenbarung, eine Implantatkomponente mit einer Oberflächenstruktur bereitzustellen, wobei die Oberflächenstruktur Hinterschneidungen aufweisen kann und die Oberflächenstruktur einschließlich der Hinterschneidungen mit einer Beschichtung beschichtet ist. Zudem war es eine Aufgabe der vorliegenden Offenbarung, ein technisches Verfahren zum Erzeugen einer Beschichtung auf dieser Oberflächenstruktur bereitzustellen. Eine weitere Aufgabe war es, dass die mit dem Verfahren erzeugte Beschichtung auf der Oberfläche möglichst durchgehend und homogen ausgebildet wird und eine antimikrobielle Wirkung aufweist.

Die beanspruchte Erfindung als Ergebnis einer oder mehrerer der sich aus dem Stand der Technik ergebenden Aufgaben wird durch die angehängten unabhängigen Ansprüche definiert. Die zugehörigen abhängigen Ansprüche definieren zudem bevorzugte Ausführungsformen.

Insbesondere waren die Erfinder des beanspruchten Gegenstands mit der Beobachtung konfrontiert, dass eine antimikrobielle Wirkung von Beschichtungen auf Implantaten mit einer Oberflächenstruktur, aber ohne Hinterschneidungen, zumindest nicht zwangsläufig auf eine Oberflächenstruktur mit Hinterschneidungen übertragbar ist. Insbesondere wurde festgestellt, dass die antimikrobielle Beschichtung die angestrebte Wirkung im Wesentlichen verliert.

Um dem entgegen zu wirken, wird eine Implantatkomponente offenbart, die einen Vollmaterialbereich und eine mit dem Vollmaterialbereich verbundene Oberflächenstruktur aufweist. Auf der Oberflächenstruktur ist eine Beschichtung vorgesehen, die neben einem At%-Anteil Ti als Hauptkomponente mindestens eine weitere Beschichtungskomponenten aufweist, wobei eine der mindestens einen weiteren Beschichtungskomponenten Silber (Ag) mit einem At%-Anteil von 15-25 At% ist. Dabei kann die Oberflächenstruktur Hinterschneidungen aufweisen, die mit dieser Beschichtung beschichtet sind.

Versuche der Erfinder haben gezeigt, dass die Anwendung einer üblicherweise verwendeten antimikrobiellen Beschichtung auf eine Implantatkomponente mit Oberflächenstruktur nicht zu der gewünschten Reduzierung von Krankheitserregern führt. Es wird vermutet, dass insbesondere Hinterschneidungen der Oberflächenstruktur zu diesem Effekt beitragen, indem sie die Wirkweise der antimikrobiellen Beschichtung beeinträchtigen.

Trotz dieser Erfahrung, konnte durch weitere Versuche dennoch überraschenderweise festgestellt werden, dass ein Silberanteil innerhalb des oben definierten Bereichs zu einer antimikrobiellen Wirkung führt. Es wird vermutet, dass hierfür eine gesteigerte Wirkung der Beschichtung im Bereich der Hinterschneidungen ursächlich sein kann. Anders ausgedrückt bewirkt die oben definierte Beschichtung vermutlich, dass die antimikrobielle Wirkung zumindest auf einem höheren Anteil der durch die Oberflächenstruktur vergrößerten Fläche der Implantatkomponente erreicht wird, und zwar insbesondere bei den Hinterschneidungen.

Die Beschichtung für die Oberflächenstruktur weist als Hauptkomponente Titan auf, da dieses Materials sich insbesondere für den Einwuchs von Knochengewebe bewährt hat. Je nach Anwendungsfall kann Titan neben Silber durch weitere Beschichtungskomponenten ergänzt werden.

Es wurde festgestellt, dass bei dem At%-Anteil von Silber insbesondere ein für eine antimikrobielle Wirkung ausreichender Silberanteil nicht nur auf den in Draufsicht sichtbaren Bereichen, sondern auch auf den in Draufsicht verdeckten Bereichen der Oberflächenstruktur, d. h. den Hinterschneidungen, vorhanden ist.

Unter einer Hinterschneidung ist dabei ein Bereich der Oberflächenstruktur zu verstehen, der bei einer Draufsicht auf eine Implantatkomponente verdeckt ist, da er sich hinter dem sichtbaren Teil der Oberflächenstruktur befindet. Eine Hinterschneidung kann zum Beispiel durch eine abgewinkelte Vertiefung oder einen abgewinkelten Vorsprung entstehen.

Derartige Hinterschneidungen werden durch das Erzeugen einer Oberflächenstruktur auf dem Vollmaterialbereich der Implantatkomponente ausgebildet. Dies kann durch eine Modifikation des Vollmaterials mittels eines Abtragens von Material an dessen Oberfläche erfolgen. Ergänzend und vorzugsweise alternativ wird die Oberflächenstruktur aber auf dem Vollmaterial aufbauend erzeugt. Anders gesagt wird auf die Oberfläche Material aufgebracht, um eine Oberflächenstruktur aufzubauen.

Die Oberflächenstruktur ist mit ihrer vergrößerten Oberfläche insbesondere für den Einwuchs von Knochengewebe vorgesehen. Dies ist vor allem bei Implantatkomponenten von Vorteil, die in das Knochengewebe integriert werden sollen, um so eine feste Verbindung mit dem Knochengewebe eines Implantationsorts einzugehen. Zum Beispiel ist eine derartige Verbindung bei Gelenkimplantaten, Gelenkversteifungen oder Knochenersatzteilen vorteilhaft.

Vorzugsweise werden die Hinterschneidungen der Oberflächenstruktur zumindest teilweise durch eine offenporige Struktur ausgebildet.

Mit anderen Worten ist die mit dem Vollmaterial verbundene Oberflächenstruktur porös. Die Oberflächenstruktur weist somit Poren auf, die offen und miteinander verbunden sind. Dementsprechend wird es Knochengewebe ermöglicht, in diese offenporige Struktur einzuwachsen. Die Poren können sich folglich zu Hohlräumen oder Verbindungskanälen ergänzen.

Eine derartige Struktur ähnelt einerseits zu einem gewissen Ausmaß einer spongiösen Knochenstruktur und fördert den Knocheneinwuchs und hat andererseits den Vorteil, dass sie als eine Art Schnittstelle zwischen dem üblicherweise sehr steifen Vollmaterial der Implantatkomponente und dem relativ dazu wesentlich weicherem Knochengewebe dient. Die Oberflächenstruktur weist somit eine strukturell bedingte geringere Steifigkeit als das Vollmaterial der Implantatkomponente auf. Auch dies kann zu einer vor allem langfristig verbesserten Integration der Implantatkomponente führen.

Bezüglich der mechanischen Widerstandsfähigkeit der Beschichtung setzt der Silberanteil zwar die Härte der Beschichtung herab, dafür wird aber die Duktilität der Beschichtung erhöht. Dies ist vor allem bei einer beschichteten porösen Oberflächenstruktur von Vorteil, da diese, wie oben beschrieben, weicher ist und sich insbesondere während der Implantation einfacher elastisch und/oder plastisch verformen kann. Die Beschichtung kann sich einer derartigen Verformung durch die Duktilität bedingt zumindest besser anpassen.

Zudem kann verhindert werden, dass das unter der Beschichtung befindliche Vollmaterial der Implantatkomponente mit Körpergewebe eines Patienten in Kontakt kommt und dabei möglicherweise zu einer Überempfindlichkeit führt. Dies ist insbesondere der Fall, wenn die Oberflächenstruktur herausgearbeitet ist oder eine aufgebrachte Oberflächenstruktur das Vollmaterial der Implantatkomponente nicht vollständig bedeckt. Dann sorgt die Beschichtung in diesem Bereich dafür, das umliegendes Gewebe nicht mit dem Vollmaterial in Kontakt kommt.

Eine Möglichkeit, eine offenporige Oberflächenstruktur zu erzeugen, ist der Einsatz einer Plasmaspraybeschichtung, insbesondere einer Titan-Plasmaspraybeschichtung. Die Verwendung einer Titan-Plasmaspraybeschichtung hat den zusätzlichen Vorteil, dass die Beschichtung der so erzeugten Oberflächenstruktur eine strapazierfähigere Verbindung eingehen kann.

Die offenporige Struktur kann ergänzend oder alternativ im Wesentlichen regelmäßig angeordneten Elementarzellen aufweisen, wobei die Elementarzellen vorzugsweise durch tetrapodenartige Grundelemente ausgebildet sind.

Durch die Regelmäßigkeit dieser offenporigen Struktur lässt sich die Steifigkeit und die Porengröße bzw. Porenweite der Oberflächenstruktur sehr gut im Voraus festlegen. Dementsprechend können sowohl Vorteile für den Knocheneinwuchs als auch in Bezug auf das Steifigkeitsverhalten realisiert werden. Als besonders geeignet haben sich hierfür Elementarzellen gezeigt, die durch tetrapodenartige Grundelemente ausgebildet werden.

Vorzugsweise weist die offenporige Struktur eine Porosität von 10 bis 80 % und/oder eine Porenweite von 45 bis 1000 µm auf.

Diese Eigenschaften beziehen sich insbesondere auf eine unbeschichtete offenporige Struktur. Sie fördern eine Beschichtung der Poreninnenflächen und damit auch der Hinterschneidungen mit einem ausreichend hohen Silberanteil. Mit anderen Worten wird das Eindringen der Silberionen während des Beschichtungsvorgangs in die Oberflächenstruktur unterstützt. Diese Werte gelten auch für Oberflächenstrukturen, die nicht unbedingt porös sind, aber Vertiefungen aufweisen, die Hinterschneidungen ausbilden.

Eine Pore der offenporigen Struktur kann in Abhängigkeit ihrer oben bereits erwähnten Ausbildung im Wesentlichen gleichförmige und gleichgroße Poren und/oder weniger gleichförmige und unterschiedliche große Poren aufweisen. Im letzteren Fall bezieht sich die Porenweite auf den weitesten Querschnitt. Dabei beträgt das Verhältnis zwischen dem weitesten und engsten Querschnitt einer Pore vorzugsweise maximal 4:1 oder 2:1. Im Allgemeinen gilt ein Verhältnis von in etwa 1 als besonders vorteilhaft.

Vorzugsweise ist die Oberflächenstruktur auf dem Vollmaterial mit einer Dicke von bis zu 4 mm, 3 mm, 2,5 mm, 2 mm, 1,5 mm, 1 mm oder 0,5 mm ausgebildet.

Hierdurch wird eine ausreichend feste Integration der Implantatkomponente im Knochengewebe sowie eine gute Versorgung des Knochengewebes erreicht.

Insbesondere beträgt der Silberanteil der Beschichtung mindestens 18 At%, 20 At% oder 22 At% und maximal 23°At%, 24 At% oder 25 At%.

In diesen Bereichen ist der Silberanteil in der Beschichtung hoch genug, sodass sich bei der Implantatkomponente eine antimikrobielle Wirkung der Oberflächenstruktur zeigt.

Besonders bevorzugt weist die Beschichtung als weitere Beschichtungskomponente einen At%-Anteil N und/oder einen At%-Anteil Nb auf.

Die Verwendung von N und/oder Nb als Beschichtungskomponenten ermöglicht eine Anpassung der Eigenschaften der Beschichtung.

In Kombination mit dem Silberanteil ergibt sich dabei nicht nur eine antimikrobielle Wirkung, sondern es ist durch Vorsehen von einer oder beider dieser Beschichtungskomponenten möglich, die Eigenschaften der Beschichtung einzustellen. Zudem beugen diese Beschichtungskomponenten gegen allergische Reaktionen von Patienten gegen das Material des Vollmaterialbereichs der Implantatkomponente vor.

Die Beschichtung liegt unabhängig von den eingesetzten Beschichtungskomponenten vorzugsweise im Wesentlichen als stöchiometrische Beschichtung vor. Eine solche Beschichtung bildet zusammen mit dem Silberanteil eine besonders gleichmäßige inerte Schicht mit antimikrobieller Wirkung aus und wirkt damit sowohl einer Überempfindlichkeit als auch einer Infektion nach Implantation des Implantats vor.

Durch einen At%-Anteil N neben der Hauptkomponente Ti und dem At%-Anteil Ag kann insbesondere eine Titannitridbeschichtung mit Silberanteil erzeugt werden (TiN-Ag).

Insbesondere können Nitrid-Beschichtungen verwendet werden, um die Abriebeigenschaften von Implantatkomponenten zu verbessern. Deswegen werden sie beispielsweise für die Laufflächen von Gelenkimplantaten verwendet, aber auch an anderen Flächen, die einer Reibkraft ausgesetzt werden, wie zum Beispiel Befestigungselemente, Klemmflächen oder Implantatoberflächen, die insbesondere bei Implantation einer Relativbewegung in Kontakt mit einer anderen Implantatkomponente oder Knochengewebe ausgesetzt sind.

Durch ihre Widerstandsfähigkeit ist die keramische Titannitridbeschichtung mit Silberanteil, d. h. Titannitrid-Silber-Beschichtung, insbesondere für strukturelle Implantate geeignet, die Teile des Skeletts unterstützen oder ersetzen, wenn sie in den Körper eines Patienten eingebracht worden sind. Hierzu gehört beispielsweise eine Implantatkomponente eines Gelenkimplantats, eines Wirbelsäulenimplantats oder auch eines Knochenimplantats, das zumindest einen Teil eines Knochens ersetzt.

Durch einen At%-Anteil Nb neben der Hauptkomponente Ti und dem At%-Anteil Ag kann insbesondere eine Titan-Niob-Beschichtung mit Silberanteil erzeugt werden (TiNb-Ag).

Die Titan-Niob-Beschichtung weist, ähnlich wie Titan, ein hohes Maß an Biokompatibilität auf. Insbesondere besitzt die Beschichtung einen niedrigen Härtegrad und ist duktiler als eine TiN-Beschichtung. Folglich kann sie sich an elastische und plastische Verformungen, die bei der Verwendung eines Implantats auftreten, besser anpassen als die TiN-Ag-Beschichtung. Genauso wie diese schützt sie ebenso effektiv vor einem Auftreten einer Überempfindlichkeit des umliegenden Gewebes.

Weiterhin ist es möglich, neben der Hauptkomponente Ti und dem At%-Anteil Ag durch Vorsehen eines At%-Anteils N und eines At%-Anteils Nb eine Titannitrid-Niob-Beschichtung mit Silberanteil zu erzeugen (TiNbN-Ag).

Ein solche Beschichtung ist sowohl gegen Abrieb widerstandsfähig als auch duktil genug, um elastischen und auch geringfügigen plastischen Verformungen standzuhalten, wie sie zum Beispiel bei der Anpassung von Knochenplatten oder Spondylodesestangen auftreten. Sie liegt mit ihren Eigenschaften damit im Wesentlichen zwischen den Eigenschaften der TiN-Ag-Beschichtung und der TiNb-Ag-Beschichtung.

Weiterhin ist es möglich die obigen Beschichtungsarten zu kombinieren, um die Eigenschaften der Beschichtung einzustellen.

Besonders bevorzugt ist die Beschichtung der Implantatkomponente eine PVD-Beschichtung ist. Das heißt, dass die Beschichtung mittels einer physikalischen Gasphasenabscheidung aufgebracht wird. Durch die Gasphasenabscheidung erreichen die ionisierten Beschichtungskomponenten etwaige Hinterschneidungen bzw. Poren der Oberflächenstruktur besonders effektiv.

Bei den obigen Beschichtungen liegt der At%-Anteil Silber in der Beschichtung vorzugsweise inselartig vor.

In Bezug auf die oben genannte Beschichtung bedeutet dies zum Beispiel, dass Ag-Inseln von Ti, TiN, TiNb und/oder TiNbN umgeben sind.

Hierdurch kann der Ag-Anteil seine infektionshemmende Wirkung besonders effektiv entfalten. Um diesem an der Oberfläche nebeneinanderliegenden Aufbau der Beschichtung und damit direkten Kontakt mit dem Gewebe oder Flüssigkeiten des Patienten zu erreichen, werden die Beschichtungskomponenten zumindest über einen Teil des Beschichtungsvorgangs gleichzeitig aufgebracht. Durch dieses gleichzeitige Aufbringen der Beschichtungskomponenten liegen die Beschichtungskomponenten zudem im Wesentlichen gleichmäßig verteilt auf der Implantatoberfläche vor.

Besonders bevorzugt weist die Beschichtung eine Dicke von 1-6 µm, 2,5-6 µm oder 3,5-5,5 µm auf.

In diesen Bereichen kann eine stabile, fortlaufende und belastbare Beschichtung auf der Oberflächenstruktur und dem, wie oben beschrieben, möglicherweise unter der Oberflächenstruktur freiliegenden Vollmaterial der Implantatkomponente erreicht werden.

Tendenziell ist eine dickere Beschichtung von Vorteil. Eine über die oben angegebenen Werte hinausgehende Dicke bringt allerdings keine nennenswerten Vorteile, sondern kann sogar eine Inhomogenität und Delamination der Beschichtung begünstigen. Es wird vermutet, dass die mechanische Widerstandsfähigkeit der Beschichtung bei den angegebenen Dicken auch dadurch bedingt ist, dass keine in der Dickenrichtung durchgehenden Abschnitte aus Silber entstehen. Mit anderen Worten liegt durch die dreidimensionale heterogene Struktur der silberhaltigen Titanbeschichtung eine im Prinzip durchgehende Beschichtung des Titans oder der Titanverbindung vor.

Weiterhin betrifft die vorliegende Offenbarung ein Verfahren zum Aufbringen einer Beschichtung auf eine Implantatkomponente, wobei das Verfahren die folgenden Schritte umfasst. Zunächst wird eine Implantatkomponente, insbesondere eine Implantatkomponenten nach einem der vorstehenden Ansprüche, bereitgestellt, wobei die Implantatkomponente mit einem Vollmaterialbereich und einer mit dem Vollmaterialbereich verbundenen Oberflächenstruktur ausgebildet ist und die Oberflächenstruktur Hinterschneidungen aufweist. Die Implantatkomponente wird in eine Beschichtungskammer eingebracht. Des Weiteren wird für das Aufbringen der Beschichtung mindestens ein Target aus einem metallischen Material bereitgestellt, das zumindest eine der Beschichtungskomponenten aufweist. Vor Beginn des Beschichtungsvorgangs wird die Beschichtungskammer geschlossen und verriegelt sowie eine Atmosphäre mit einem Druck von 0,001 bis 0,01 mbar bereitgestellt. Die Atmosphäre kann dabei eine Beschichtungskomponente aufweisen. Nach dem Bereitstellen der Atmosphäre erfolgt das Zünden eines Lichtbogens zum Verdampfen des metallischen Materials des mindestens einen Targets und das Beschichten der Oberflächenstruktur (und möglicherweise eines freiliegenden Bereichs des Vollmaterials unter der Oberflächenstruktur) mit dem verdampften metallischen Material des mindestens einen Targets

Dieses Verfahren ermöglicht eine gleichzeitige und vor allem durchgehende Beschichtung der Oberflächenstruktur innerhalb der Beschichtungskammer. Dabei wird durch das gleichzeitige Beschichten und den Silberanteil sichergestellt, dass an der Oberfläche der Beschichtung Silber exponiert ist und sich damit der infektionshemmende Effekt der Beschichtung entfalten kann. Dies gilt auch für etwaige Hinterschneidungen bzw. Poren. Zudem kann durch eine Wahl der Anzahl der jeweiligen Targets der Beschichtungskomponenten der At%-Anteil zumindest in seiner Größenordnung auf die gewünschte Zusammensetzung der Beschichtung eingestellt werden.

Alternativ oder zusätzlich zu der Einstellung der Zusammensetzung der Beschichtung über die Anzahl der jeweiligen Targets kann mindestens ein Target, das ein vorbestimmtes Verhältnis von mindesten zwei Beschichtungskomponenten aufweist, bereitgestellt werden. Insbesondere weist das mindestens eine Target Silber, Titan und/oder Niob auf.

Alternativ zu dem obigen Druckbereich kann der Druck der Atmosphäre für den Beschichtungsvorgang auch auf einen Druckbereich von 0,003 mbar oder 0,005 mbar bis 0,1 mbar, 0,05 mbar, 0,02 mbar, 0,015 mbar oder 0,01 mbar eingestellt werden. Der höhere Druckbereich hat den Vorteil, dass hiermit die Beschichtungskomponenten insbesondere die Hinterschneidungen bzw. Poren besser erreichen und sorgt somit für eine höhere und gleichmäßigere Beschichtungsdicke.

Des weiteren kann im Rahmen der Bereitstellung der Implantatkomponente die Oberflächenstruktur durch miteinander verschmolzene Partikeln ausgebildet werden, die mittels eines Plasmasprayverfahrens, insbesondere mittels eines Titan-Plasmasprayverfahren, auf das Vollmaterial der Implantatkomponente aufgebracht wird.

Eine derartige Oberflächenstruktur fördert durch ihre Hinterschneidungen und/oder Porosität den Einwuchs von Knochengewebe und damit für eine stabile Integration der Implantatkomponente.

Besonders bevorzugt wird die Oberflächenstruktur aus Elementarzellen aufgebaut und vorzugsweise mittels eines Schichtschmelzverfahrens, insbesondere eines Elektronenstrahl-Schichtschmelzverfahrens ausgebildet.

Auch diese Oberflächenstruktur fördert den Einwuchs von Knochengewebe und bildet zudem, wie oben beschrieben, eine im Wesentlichen regelmäßige Oberflächenstruktur aus, deren Eigenschaften durch die Größe der Elementarzellen bzw. der diesen zugrunde liegenden tetrapodenartigen Grundelemente eingestellt werden können. Zudem begünstigt die Regelmäßigkeit der Struktur auch eine gleichmäßige Beschichtung der Oberflächenstruktur.

### AUSFÜHRLICHEN ERLÄUTERUNG BEVORZUGTER AUSFÜHRUNGSFORMEN

Unter einer Beschichtung ist im Rahmen der vorliegenden Erfindung eine durch ein technisches Verfahren aufgebrachte Beschichtung zu verstehen. Beispiele solcher technischer Verfahren sind die Gasphasenabscheidung (CVD - Chemical Vapor Deposition oder PVD - Physical Vapor Deposition) oder auch Plasmasprühverfahren, wie insbesondere das oben bereits aufgeführte Titan-Plasmasprayverfahren.

Wie oben beschrieben umfasst eine erfindungsgemäße Beschichtung Titan und Silber sowie vorzugsweise bis zu zwei weitere Beschichtungskomponenten. Der Einfachheit halber wird eine derartige Beschichtung im Folgenden als Titan-Silber-Beschichtung bezeichnet, mit dem Verständnis, dass diese auch weitere Beschichtungskomponenten umfassen kann. Zudem wird angemerkt, dass die Beschichtung durch den technischen Beschichtungsvorgang bis zu einem Anteil von 3 %, 2 % oder 1 % Verunreinigungen in Form anderer Elemente oder Verbindungen enthalten kann. Dies gilt insbesondere auch für eine Beschichtung, die aus bestimmten Beschichtungskomponenten besteht.

Der Auftrag der Beschichtung erfolgt auf einer Oberflächenstruktur der Implantatkomponente. Ein bevorzugtes Verfahren, mit dem eine derartige Oberflächenstruktur erzeugt werden kann, ist ein Plasmasprayverfahren. Bei dem Plasmasprayverfahren wird im Wesentlichen in einem Vakuum ein Plasmastrahl erzeugt, dem Metallpartikel zugeführt werden, die in dem hochenergetischen Strahl schmelzen und beschleunigt werden. Anschließend treffen die geschmolzenen Metallpartikel auf die Oberfläche der Implantatkomponente und verbinden sich mit dieser. Die Metallpartikel weisen vorzugsweise Titan oder eine Titanlegierung auf oder bestehen im Wesentlichen daraus.

Eine durch das Plasmasprayverfahren erzeugte Oberflächenstruktur weist vorzugsweise eine Rauheit (Ra) in einem Bereich von 20 µm bis 80 µm auf. Zudem weist eine mit diesem Verfahren erzeugte Oberflächenstruktur insbesondere Hinterschneidungen auf.

Es wurde festgestellt, dass diese Hinterschneidungen mit einer üblicherweise verwendeten antimikrobiellen Beschichtung nicht so ausreichend beschichtet werden, dass sich die gewünschte Wirkung einstellt. Es wird nun vermutet, dass dieses insbesondere auf einen geringeren Silberanteil in diesem Bereich zurückzuführen ist.

Zudem ist eine mit einem Plasmasprayverfahren erzeugte Oberflächenstruktur vorzugsweise porös. Die Porosität liegt bevorzugt in einem Bereich von 10 % bis 60 % und noch bevorzugter in einem Bereich von 15 % bis 60 % oder in einem Bereich von 20 % bis 50 % (gemessen nach ASTM F1854).

In diesen Bereichen kann zudem eine ausreichende Porenweite für den Einwuchs von Gewebe, insbesondere von Knochengewebe, vorgesehen werden. So weist die Oberflächenstruktur insbesondere Porenweiten in einem Bereich von in etwa 45 µm bis 80 µm und vorzugsweise in einem Bereich von 50 µm bis 70 µm auf. Auch die durchschnittliche Porenweite sämtlicher Poren liegt vorzugsweise in diesem Bereich (arithmetischer Mittelwert).

Ein anderes, besonders bevorzugtes Verfahren erzeugt die Oberflächenstruktur mittels eines additiven Verfahrens. Insbesondere eignen sich hierfür Schichtschmelzverfahren, wie zum Beispiel das oben bereits erwähnte Elektronenstrahl-Schichtschmelzverfahren. Der Vorteil von additiven Verfahren ist, dass die Oberflächenstruktur definiert und regelmäßig auf der Implantatkomponente ausgebildet werden kann. Besonders bevorzugt wird bei einem solchen additiven Verfahren das Vollmaterial und die Oberflächenstruktur der Implantatkomponente im Wesentlichen im gleichen Herstellungsschritt hergestellt.

Wie oben dargelegt, weist die Oberflächenstruktur vorzugsweise Elementarzellen auf, aus denen sie aufgebaut wird. Noch bevorzugter sind die Elementarzellen wiederum aus mindestens einem und insbesondere genau einem Grundelementtyp aufgebaut, wie zum Beispiel einem tetrapodenartigen Grundelement. Diesbezüglich wird insbesondere auf die in der Patentanmeldung WO 2017/005514 A1 offenbarten porösen Strukturen verwiesen.

Ein Vorteil einer mit einem additiven Verfahren hergestellten porösen Struktur ist, dass sowohl die Porosität, Porenweite, Porengröße und/oder die Porenform regelmäßig hergestellt werden können. Zudem ermöglicht die Ausbildung der Oberflächenstruktur vorbestimmte mechanische Eigenschaften, wie zum Beispiel eine (richtungsabhängige) Elastizität. So kann die Oberflächenstruktur insbesondere für ein bestimmtes Gewebe eingerichtet werden, wie zum Beispiel Knochengewebe oder Bindegewebe.

Auch ist durch ein additives Verfahren eine vordefinierte und insbesondere auch höhere Porosität der Oberflächenstruktur herstellbar. So beträgt die Porosität bevorzugt von 50 % bis 80 % und noch bevorzugter 65 % bis 75 % (gemessen nach ASTM F1854).

Weiterhin kann bei einem additiven Verfahren auf einfache Weise eine ausreichende Porenweite für den Einwuchs von Gewebe, insbesondere von Knochengewebe, bereitgestellt werden. So weist die Oberflächenstruktur insbesondere Porenweiten in einem Bereich von in etwa 100 µm bis 1000 µm, vorzugsweise in einem Bereich von 300 µm bis 900 µm und noch bevorzugter in einem Bereich von 500 µm bis 800 µm auf. Durch die Regelmäßigkeit der Struktur wird die durchschnittliche Porenweite im Wesentlichen ebenfalls vorbestimmt und weicht herstellungsbedingt nur wenig von dem Sollmaß ab (z. B. kann der arithmetische Mittelwert weniger als 50 µm abweichen) . Mit anderen Worten ist die Varianz bei additiven Verfahren geringer als bei anderen Verfahren, wie zum Beispiel bei dem oben erwähnten Plasmasprayverfahren.

Die bei den obigen Verfahren erzeugten Porengrößen weisen vorzugsweise ein Verhältnis zwischen breitester und schmalster Porenweite auf, das geringer als 4:1, 3:1 und noch bevorzugter 2:1 ist.

Die Beschichtung der Oberflächenstruktur weist eine, zwei oder drei Schichten der Beschichtung auf, in der Silber in eingebetteter Form vorliegt. Insbesondere liegt das Silber in Form von Silberinseln (Silberagglomeraten) vor, d. h. Silber beziehungsweise Silberatome sind neben dem Gitter der restlichen Beschichtungskomponenten angeordnet. Aufgrund der Größe der Silberatome wird davon ausgegangen, dass, wenn überhaupt, nur ein geringer Anteil des Silbers interstitiell in dem Gitter angeordnet ist.

Insbesondere wurde beobachtet, dass das Silber in Form von Silberagglomeraten in der Titan-Silber-Beschichtung vorliegt. Mit anderen Worten liegt das Silber in dem Gitter der Beschichtung vor, ist aber nicht in dieses integriert. Die Silberagglomerate liegen vorzugsweise in einem Bereich von 1 µm bis 50 µm und noch bevorzugter in einem Bereich von 5 µm bis 30 µm vor.

Des Weiteren wird davon ausgegangen, dass die Wirksamkeit des Silbers insbesondere dadurch entsteht, dass es im implantierten Zustand der Implantatkomponente bei Kontakt mit Körperflüssigkeit durch Lokalelementbildung in den ionischen Zustand übergeht und so seine antimikrobielle Wirkung entfaltet. Dass diese Lokalelementbildung stattfinden kann, wird durch eine Anordnung dieser Inseln an der Oberfläche der Beschichtung auf sehr effektive Weise ermöglicht. Erreicht wird diese Anordnung durch ein zumindest partiell gleichzeitiges Beschichten der Implantatkomponente mit Titannitrid und Silber.

Die Beschichtung weist durch ihre antimikrobiellen Eigenschaften eine infektionshemmende Wirkung auf. Es wird vermutet, dass der vorliegende Silberanteil der Beschichtung die Ausbildung eines Biofilms stört, den diese Bakterien entwickeln. Durch diese Störung funktioniert dann der durch diesen Biofilm hervorgerufene Schutz für die Bakterien nicht mehr ausreichend, sodass sie durch das Immunsystem von Patienten oder auch durch Wirkstoffe besser angreifbar sind.

Weiterhin wurde beobachtet, dass sich das Silber in Ionenform aus der Beschichtung lösen kann. Es wird davon ausgegangen, dass diese an der Oberfläche der Beschichtung ionisierten Silberpartikel in unmittelbarer Umgebung der Implantatkomponente eine Wirkzone ("inhibition zone") ausbilden, in der sie eine antimikrobielle Wirkung entfalten. Folglich kann mit der Beschichtung nicht nur gegen eine sich unmittelbar von der Oberfläche der Implantatkomponente aus ausbreitenden Infektion vorgebeugt werden, sondern insbesondere auch gegen eine Infektion in dem an der Implantatkomponente anliegenden Gewebe eines Patienten.

Vorzugsweise ist der At%-Anteil an Silber geringer als der At%-Anteil an Titan. Mit anderen Worten ist es nicht erforderlich, dass eine stöchiometrische Verteilung vorliegt. Die Verteilung kann über- oder unterstöchiometrisch sein.

Der Silberanteil führt zusammen mit dem Anteil von Titan und möglichen weiteren Beschichtungskomponenten neben der oben erwähnten antimikrobiellen Wirkung zudem zu einer Veränderung der mechanischen Eigenschaften im Verhältnis zu einer Beschichtung ohne Silberanteil. Insbesondere wird die Beschichtung durch diesen Aufbau weicher und duktiler.

Es wird vermutet, dass aufgrund einer damit einhergehenden Duktilität die mechanische Widerstandsfähigkeit bzw. Festigkeit der Beschichtung weiter ausreicht, um den bei einer Implantation der Implantatkomponente auftretenden mechanischen Einflüssen zu widerstehen. Solche mechanischen Einflüsse entstehen zum Beispiel beim Erzeugen eines Presssitzes einer Implantatkomponente im Knochengewebe, durch Kontakt einer Implantatkomponente mit einem Befestigungselement, wie zum Beispiel beim Eindrehen von Knochenschrauben zur Befestigung einer Platte, oder bei der Montage mit einer anderen Implantatkomponente, wie zum Beispiel bei einem Spondylodeseaufbau.

Aus diesem Grund ist die vorliegende Beschichtung insbesondere für Implantatkomponenten geeignet, welche nach Implantation das Skelett eines Patienten unterstützen oder Teile dieses Skeletts ersetzen. Bei solchen Implantatkomponenten tritt eine mechanische Belastung der Beschichtung im Allgemeinen während der Implantation und des Zusammenbaus mehrerer Implantatkomponenten auf. Nach Implantation entstehen insbesondere durch die alltägliche Belastung der Implantatkomponente im Körper eines Patienten Spannungen und Dehnungen in der Beschichtung aufgrund elastischer Verformungen. Auch hier ist die vorliegende Beschichtung von Vorteil, da sie diesen standhält ohne ihre schützende Wirkung zu verlieren.

Wie oben beschrieben können die Beschichtungskomponenten so gewählt werden, dass die Beschichtung vor allem für Implantatkomponenten geeignet ist, bei denen Abrieb vor allem während der Implantation der Implantatkomponente und/oder dem Zusammenbau mehrerer Implantatkomponenten besteht. Es wurde festgestellt, dass hierfür eine Dicke der Beschichtung unter 10 µm, insbesondere von 2,5-6 µm, bevorzugt 3,5-5,5 µm und noch bevorzugter in etwa 4,5 µm, ausreichend ist. Dies gilt auch für die oben beschriebene TiNb-Ag Beschichtung, die eine besonders hohe Anpassungsfähigkeit an eine Verformung der Oberflächenstruktur zeigt.

Weiterhin kann bei der vorliegenden Beschichtung durch den Silberanteil teilweise ein Unterschied ihrer Materialeigenschaften, insbesondere der Elastizität, zu dem darunterliegenden Material der Oberflächenstruktur verringert werden. Auch dadurch können eine ausreichende mechanische Widerstandsfähigkeit und Haftung der Beschichtung begünstigt werden.

Zusammenfassend zeigt die vorliegende Beschichtung für die Oberflächenstruktur einer Implantatkomponente somit sowohl vorteilhafte antimikrobielle als auch vorteilhafte mechanische Eigenschaften, die für eine mit dieser Beschichtung zumindest abschnittsweise beschichteten Implantatkomponente von Nutzen sind.

Vorzugsweise wird eine derartige Beschichtung durch die oben bereits genannte physikalische Gasabscheidung (PVD-Verfahren) hergestellt. Vor dem Einbringen in die Beschichtungskammer kann die mit der Beschichtung zu versehende Implantatkomponente einschließlich ihrer Oberflächenstruktur wässrig gereinigt werden.

Dann wird die Implantatkomponente in die Beschichtungskammer eingeführt, die anschließend evakuiert wird. Für die nachfolgenden Prozesse wird die Implantatkomponente vorzugsweise auf 400 bis 600 °C aufgeheizt, um die Beweglichkeit von Ionen an der Oberflächenstruktur der Implantatkomponente zu verbessern und damit eine bessere Haftung und Verteilung der Beschichtung auf der Oberflächenstruktur und einer möglicherweise darunterliegenden zu beschichtenden Oberfläche des Vollmaterials zu erreichen.

Weiterhin ist es möglich, eine Reinigung der Implantatoberfläche mittels Ionenätzens durchzuführen. Hierbei wird die Implantatkomponente unter einer inerten Atmosphäre, insbesondere einer Argonatmosphäre, mit Ionen (z. B. Titanionen, Argonionen) beschossen, um eine an der Oberfläche der unbeschichteten Oberflächenstruktur möglicherweise vorhandene Oxidschicht zu entfernen. Auch hierdurch wird eine bessere Haftung der Beschichtung an der Oberflächenstruktur der Implantatkomponente erreicht.

Nach dieser optionalen Reinigung erfolgt das Aufbringen der Beschichtung auf die Implantatkomponente unter einer Atmosphäre. Bei der Atmosphäre wird für den Fall der Verwendung von Stickstoff als Beschichtungskomponente eine Atmosphäre verwendet die zumindest Stickstoff enthält. Ansonsten erfolgt die Beschichtung unter einer Atmosphäre, die im Wesentlichen aus einem inerten Gas besteht.

Wie oben bereits beschrieben, kann diese Beschichtung entsprechend ihrer gewünschten Zusammensetzung mit mindestens einem Silbertarget, mindestens einem Titantarget sowie mindestens einem weiteren Target mit einer weiteren Beschichtungskomponente ausgeführt werden. Genauso ist es möglich, ein oder mehrere Targets zu verwenden, das die für die Beschichtung vorgesehenen At%-Anteile an Silber, Titan und möglicherweise weiterer Beschichtungskomponenten aufweist. Hierbei wird folglich die Zusammensetzung der Beschichtung insbesondere durch die Zusammensetzung des mindestens einen Targets bestimmt.

Um eine Streuung des verdampften Targetmaterials an Gasteilchen in der Beschichtungskammer hervorzurufen, sodass diese besser zu den Oberflächen von Hinterschneidungen bzw. Poren gelangen, erfolgt die Beschichtung unter den oben aufgeführten Unterdruckbereichen.

Ist die gewünschte Atmosphäre eingestellt, beginnt der Verdampfungsprozess des mindestens einen Targets. Besonders bevorzugt wird hierfür ein Lichtbogen verwendet, der durch einen starken Strom mittels elektrischer Entladung Material aus den Targets herauslöst und in die Gasphase überführt. Bei dieser Entladung werden insbesondere Spannungen in einem Bereich von 15-30 V und bevorzugt in einem Bereich von 20-25 V sowie Ströme in einem Bereich von 40-70 A eingesetzt. Dem Fachmann ist es allerdings verständlich, dass auch andere Verfahren zum Verdampfen der Targets verwendet werden können, wie zum Beispiel thermisches Verdampfen, Elektronenstrahl-verdampfen oder Laserstrahlverdampfen.

Zumindest während eines Teils des Beschichtungsvorgangs erfolgt bei Verwendung von Targets mit unterschiedlichen Materialien die Beschichtung gleichzeitig, um die oben beschriebene Ag-Inselstruktur zu erzeugen.

Je nach zu beschichtendem Material der Oberflächenstruktur kann an diesem zudem eine negative Spannung von 100 V bis 1500 V angelegt werden, um die Haftung und Schichthomogenität zu verbessern. Auch können, um eine möglichst gleichmäßige Beschichtung der Oberflächenstruktur zu erreichen, die Targets und die Implantatkomponente während des Beschichtungsvorgangs relativ zueinander bewegt werden.

Nach erfolgter Beschichtung und einer Abkühlphase wird die Beschichtungskammer wieder belüftet und die beschichtete Implantatkomponente kann entnommen werden. Die Abkühlung erfolgt vorzugsweise mit Unterstützung einer Gasatmosphäre (z. B. Stickstoff oder ein inertes Gas) für eine verbesserte Wärmeabfuhr, sodass der Abkühlungsvorgang beschleunigt wird.

## Patentansprüche

1. Implantatkomponente, die aufweist:
einen Vollmaterialbereich;
eine mit dem Vollmaterialbereich verbundene Oberflächenstruktur, welche für den Einwuchs von Knochengewebe vorgesehen ist;
eine Beschichtung, die auf der Oberflächenstruktur vorgesehen ist, **dadurch gekennzeichnet, dass** die Beschichtung als Hauptkomponente einen At%-Anteil Ti und als eine weitere Beschichtungskomponente Ag mit einem At%-Anteil von 15-25 At% aufweist; und
wobei ein überwiegender Anteil des Silbers in Form von Silberagglomeraten neben einem Gitter der restlichen Beschichtungskomponenten angeordnet ist, wobei wenn überhaupt, nur ein geringer Anteil des Silbers interstitiell in dem Gitter angeordnet ist,
wobei die Oberflächenstruktur eine offenporige Struktur ist, die mittels einer Plasmaspraybeschichtung, insbesondere einer Titan-Plasmaspraybeschichtung, erzeugt worden ist.

2. Implantatkomponente nach Anspruch 1, bei der die Oberflächenstruktur Hinterschneidungen aufweist, die mit der Beschichtung beschichtet sind.

3. Implantatkomponente nach Anspruch 1 oder 2, bei der die Oberflächenstruktur eine Rauheit (Ra) in einem Bereich von 20 µm bis 80 µm aufweist.

4. Implantatkomponente nach einem der vorstehenden Ansprüche, bei der die offenporige Struktur im Wesentlichen regelmäßig angeordneten Elementarzellen aufweist und die Elementarzellen vorzugsweise durch tetrapodenartige Grundelemente ausgebildet sein.

5. Implantatkomponente nach einem der Ansprüche 2 bis 4, bei der die offenporige Struktur eine Porosität von 10 % bis 80 % und/oder eine Porenweite von 45 bis 1000 µm auf.

6. Implantatkomponente nach einem der vorstehenden Ansprüche, bei der die Oberflächenstruktur mit einer Dicke von bis zu 4 mm, 3 mm, 2,5 mm, 2 mm, 1,5 mm, 1 mm oder 0,5 mm ausgebildet ist.

7. Implantatkomponente nach einem der vorstehenden Ansprüche, bei welcher der Silberanteil der Beschichtung mindestens 18 At%, 20 At% oder 22 At% und maximal 23 At%, 24 At% oder 25 At% beträgt.

8. Implantatkomponente nach einem der vorstehenden Ansprüche, bei der die Beschichtung als weitere Beschichtungskomponente einen At%-Anteil N.

9. Implantatkomponente nach einem der vorstehenden Ansprüche, bei der ein Anteil der Silberagglomerate an der Oberfläche der Beschichtung exponiert ist.

10. Implantatkomponente nach einem der vorstehenden Ansprüche, wobei die Silberagglomerate eine Partikelgröße in einem Bereich von 1 µm bis 50 µm und bevorzugt in einem Bereich von 5 µm bis 30 µm aufweisen.

11. Implantatkomponente nach einem der vorstehenden Ansprüche, bei der die Beschichtung eine Dicke von 1-6 µm, 2,5-6 µm oder 3,5-5,5 µm aufweist.

12. Implantatkomponente nach einem der vorstehenden Ansprüche, bei welcher der Vollmaterialbereich eine Legierung aufweist oder aus dieser besteht und die Legierung vorzugsweise Titan als Hauptlegierungskomponente aufweist.

13. Verfahren zum Aufbringen einer Beschichtung auf eine Implantatkomponente, wobei das Verfahren die Schritte umfasst:
- Bereitstellen einer Implantatkomponente nach einem der vorstehenden Ansprüche, wobei die Implantatkomponente mit einem Vollmaterialbereich und einer mit dem Vollmaterialbereich verbundenen Oberflächenstruktur ausgebildet ist, welche für den Einwuchs von Knochengewebe vorgesehen ist;;
- Einbringen der Implantatkomponente in eine Beschichtungskammer;
- Bereitstellen von mindestens einem Target aus einem metallischen Material, das zumindest eine der Beschichtungskomponenten aufweist;
- Verriegeln der Beschichtungskammer;
- Bereitstellen einer Atmosphäre mit einem Druck von 0,001 bis 0,01 mbar;
- Zünden eines Lichtbogens zum Verdampfen des metallischen Materials des mindestens einen Targets; und
- Beschichten der Oberflächenstruktur mit dem verdampften metallischen Material des mindestens einen Targets, wobei die Beschichtung als Hauptkomponente einen At%-Anteil Ti und als eine weitere Beschichtungskomponente Ag mit einem At%-Anteil von 15-25 At% aufweist wobei ein überwiegender Anteil des Silbers in Form von Silberagglomeraten neben einem Gitter der restlichen Beschichtungskomponenten angeordnet ist, wobei wenn überhaupt, nur ein geringer Anteil des Silbers interstitiell in dem Gitter angeordnet ist,
bei dem die Oberflächenstruktur durch miteinander verschmolzene Partikeln ausgebildet wird, die mittels eines Plasmasprayverfahrens, insbesondere mittels eines Titan-Plasmasprayverfahren, auf das Vollmaterial der Implantatkomponente aufgebracht wird.

14. Verfahren nach Anspruch 13, bei dem die Oberflächenstruktur eine Rauheit (Ra) in einem Bereich von 20 µm bis 80 µm aufweist.

15. Verfahren nach Anspruch 13 oder 14, bei dem ein Anteil der Silberagglomerate an der Oberfläche der Beschichtung exponiert wird.

## Claims

1. An implant component, having:
a solid material region;
a surface structure connected to said solid material region, which surface structure is provided for the ingrowth of bone tissue;
a coating which is provided on the surface structure, **characterized in that** the coating has as its main component an atomic percentage of Ti and as a further coating component Ag with an atomic percentage of 15-25 at%; and
wherein a predominant proportion of the silver is arranged in the form of silver agglomerates next to a lattice of the remaining coating components, wherein only a small proportion, if any, of the silver is arranged interstitially in the lattice,
wherein the surface structure is an open-pore structure which was produced by means of a plasma spray coating, in particular a titanium plasma spray coating.

2. The implant component according to claim 1, wherein the surface structure has undercuts which are coated with the coating.

3. The implant component according to claim 1 or 2, wherein the surface structure has a roughness (Ra) in a range of 20 µm to 80 µm.

4. The implant component according to any one of the preceding claims, wherein the open-pore structure comprises substantially regularly arranged unit cells and the unit cells are preferably formed as tetrapod-like basic elements.

5. The implant component according to any one of claims 2 to 4, wherein the open-pore structure has a porosity of 10% to 80% and/or a pore width of 45 to 1000 mm.

6. The implant component according to any one of the preceding claims, wherein the surface structure is formed with a thickness of up to 4 mm, 3 mm, 2.5 mm, 2 mm, 1.5 mm, 1 mm, or 0.5 mm.

7. The implant component according to any one of the preceding claims, wherein the silver proportion of the coating is at least 18 at%, 20 at%, or 22 at% and at most 23 at%, 24 at%, or 25 at%.

8. The implant component according to any one of the preceding claims, wherein the coating comprises an atomic percentage of N as a further coating component.

9. The implant component according to any one of the preceding claims, wherein a proportion of the silver agglomerates is exposed on the surface of the coating.

10. The implant component according to any one of the preceding claims, wherein the silver agglomerates are present in a range from 1 µm to 50 µm, preferably from 5 µm to 30 µm.

11. The implant component according to any one of the preceding claims, wherein the coating has a thickness of 1-6 µm, 2.5-6 µm, or 3.5-5.5 µm.

12. The implant component according to any one of the preceding claims, wherein the solid material region has or consists of an alloy and the alloy preferably has titanium as its main alloy component.

13. A method for applying a coating onto an implant component, wherein the method comprises the steps of:
- providing an implant component, in particular an implant component according to any one of the preceding claims, wherein the implant component is formed with a solid material region and with a surface structure connected to the solid material region, which is provided for the ingrowth of bone tissue;
- introducing the implant component into a coating chamber;
- providing at least one target made of a metallic material which has at least one of the coating components;
- locking the coating chamber;
- providing an atmosphere with a pressure of 0.001 to 0.01 mbar;
- igniting an electric arc for evaporation of the metallic material of the at least one target; and
- coating the surface structure with the evaporated metallic material of the at least one target, wherein the coating, as a main component, has an atomic percentage of Ti and as a further coating component Ag with an atomic percentage of 15-25 at% and an atomic percentage of Nb, wherein a predominant proportion of the silver is arranged in the form of silver agglomerates next to a lattice of the remaining coating components, wherein only a small proportion, if any, of the silver is arranged interstitially in the lattice,
wherein the surface structure is formed by fused particles, which surface structure is applied onto the solid material of the implant component by means of a plasma spraying method, in particular by means of a titanium plasma spraying method.

14. The method according to claim 13, wherein the surface structure has a roughness (Ra) in a range of 20 µm to 80 µm.

15. The method according to claim 13 or 14, wherein a proportion of the silver agglomerates is exposed at the surface of the coating.

## Revendications

1. Composant d'implant, comprenant :
une zone de matériau solide ;
une structure de surface reliée à la zone de matériau solide, prévue pour la croissance du tissu osseux ;
un revêtement prévu sur la structure de surface, **caractérisé en ce que** le revêtement présente, comme composant principal, une proportion en % at de Ti et comme autre composant de revêtement de l'Ag à raison d'une proportion en % at de 15 à 25 % ; et
dans lequel une proportion prédominante d'argent est disposée sous forme d'agglomérats d'argent à proximité d'un réseau des autres composants du revêtement, dans lequel, le cas échéant, seule une petite proportion de l'argent est disposée de manière interstitielle dans le réseau,
dans lequel la structure de surface est un structure à pores ouverts qui a été réalisée au moyen d'un revêtement par pulvérisation plasma, en particulier d'un revêtement par pulvérisation plasma de titane.

2. Composant d'implant selon la revendication 1, dans lequel la structure de surface présente des contre-dépouilles qui sont recouvertes par le revêtement.

3. Composant d'implant selon la revendication 1 ou 2, dans lequel la structure de surface présente une rugosité (Ra) dans une plage comprise entre 20 µm et 80 µm.

4. Composant d'implant selon l'une quelconque des revendications précédentes, dans lequel la structure à pores ouverts présente des cellules élémentaires disposées de manière essentiellement régulière et les cellules élémentaires sont de préférence formées par des éléments de base de type tétrapode.

5. Composant d'implant selon l'une des revendications 2 à 4, dans lequel la structure à pores ouverts présente une porosité de 10 % à 80 % et/ou une taille de pores de 45 à 1000 µm.

6. Composant d'implant selon l'une quelconque des revendications précédentes, dans lequel la structure de surface est formée à raison d'une épaisseur allant jusqu'à 4 mm, 3 mm, 2,5 mm, 2 mm, 1,5 mm, 1 mm ou 0,5 mm.

7. Composant d'implant selon l'une quelconque des revendications précédentes, dans lequel la proportion en argent du revêtement est d'au moins 18 % at, 20 % at ou 22 % at et d'au plus 23 % at, 24 % at ou 25 % at.

8. Composant d'implant selon l'une quelconque des revendications précédentes, dans lequel le revêtement présente, en tant que composant de revêtement supplémentaire, une proportion en % at de N.

9. Composant d'implant selon l'une quelconque des revendications précédentes, dans lequel une proportion des agglomérats d'argent est exposée à la surface du revêtement.

10. Composant d'implant selon l'une quelconque des revendications précédentes, dans lequel les agglomérats d'argent présentent une taille de particules dans une plage comprise entre 1 µm et 50 µm, et de préférence dans une plage comprise entre 5 µm et 30 µm.

11. Composant d'implant selon l'une quelconque des revendications précédentes, dans lequel le revêtement présente une épaisseur de 1 à 6 µm, de 2,5 à 6 µm ou de 3,5 à 5,5 µm.

12. Composant d'implant selon l'une quelconque des revendications précédentes, dans lequel la zone de matériau solide présente un alliage ou est constituée d'un alliage et l'alliage présente de préférence du titane comme composant d'alliage principal.

13. Procédé pour appliquer un revêtement sur un composant d'implant, dans lequel le procédé comprend les étapes :
- de fourniture d'un composant d'implant selon l'une quelconque des revendications précédentes, dans lequel le composant d'implant est réalisé avec une zone de matériau solide et une structure de surface reliée à la zone de matériau solide, prévue pour la croissance du tissu osseux ;
- d'introduction du composant d'implant dans une chambre de revêtement ;
- de fourniture d'au moins une cible d'un matériau métallique qui présente au moins l'un des composants de revêtement ;
- de verrouillage de la chambre de revêtement ;
- de fourniture d'une atmosphère à une pression comprise entre 0,001 et 0,01 mbar ;
- d'amorçage d'un arc électrique pour vaporiser le matériau métallique de l'au moins une cible ; et
- de revêtement de la structure de surface avec le matériau métallique vaporisé de l'au moins une cible, dans lequel le revêtement présente, comme composant principal, une proportion en % at de Ti et, comme autre composant de revêtement, de l'Ag à raison d'une proportion en % at de 15 à 25 % at, dans lequel une proportion prédominante d'argent est disposée sous forme d'agglomérats d'argent à proximité d'un réseau des autres composants du revêtement, dans lequel, le cas échéant, seule une petite proportion de l'argent est disposée de manière interstitielle dans le réseau, dans lequel la structure de surface est formée de particules fusionnées entre elles, qui sont appliquées sur le matériau solide du composant d'implant au moyen d'un procédé de pulvérisation plasma, en particulier au moyen d'un procédé de pulvérisation plasma de titane.

14. Procédé selon la revendication 13, dans lequel la structure de surface présente une rugosité (Ra) dans une plage comprise entre 20 µm et 80 µm.

15. Procédé selon la revendication 13 ou 14, dans lequel une proportion des agglomérats d'argent est exposée à la surface du revêtement.
